# EUROPÄISCHE PATENTSCHRIFT

(11) **EP 0 675 129 B1**
(45) Veröffentlichungstag und Bekanntmachung des Hinweises auf die Patenterteilung: **09.08.2000**
(21) Anmeldenummer: 95103819.9
(22) Anmeldetag: 16.03.1995
(51) Int. Cl.: C07F 9/40, C07C 309/68, C07C 317/18, C07C 395/00, C07C 391/00, C07F 7/02, C07C 217/40, C07C 41/48

(54) **Verfahren zur Herstellung von heterosubstituierten Acetalen**
Process for preparing hetero substituted acetals
Procédé de préparation d'acétals hétéro substitués

(30) Priorität: 29.03.1994 DE 4410867
(43) Veröffentlichungstag der Anmeldung: 04.10.1995
(73) Patentinhaber: BAYER AG, 51368 Leverkusen (DE)
(72) Erfinder: Landscheidt, Heinz, Dr., D-47057 Duisburg (DE); Klausener, Alexander, Dr., D-50670 Köln (DE)

(56) Entgegenhaltungen:
- EP-A- 0 055 108
- EP-A- 0 359 118
- JOURNAL OF HETEROCYCLIC CHEMISTRY, Bd.29, 1992, PROVO US Seiten 1625 - 1629 TAEKO IZUMI 'Palladium-catalyzed synthesis of indoles from 2-nitrostyrenes'
- SYNTHESIS (SYNTBF,00397881);90; (8); PP.694, YAMAGUCHI UNIV.;FAC. LIB. ARTS; YAMAGUCHI; 753; JAPAN (JP) KAMIMURA A ET AL 'Facile preparation of.alpha.-nitroaldehyde acetals from (E/Z)-2-nitro-1-(phenylthio)-1-alkenes'
- ZH. ORG. KHIM. (ZORKAE,05147492);84; VOL.20 (10); PP.2114-18, IRKUTSK. GOS. UNIV.;INST. NEFTE-UGLEKHIM. SINT.; IRKUTSK; USSR (SU) BYCHKOVA T I ET AL 'Reaction of 2-phenoxyethenyl alkyl- or aryl sulfones with sodium diethyldithiocarbamate'
- J. CHEM. SOC., PERKIN TRANS. 2 (JCPKBH);76; (11); PP.1210-13, WEIZMANN INST. SCI.;REHOVOT; ISRAEL HALMANN M ET AL 'Reaction of phosphonated acetals. Acid-catalyzed hydrolysis of dialkyl 2,2-dialkoxyethylphosphonates'
- SYNTHESIS (SYNTBF,00397881);87; (7); PP.633-5, INST. ORG. PHARM. CHEM.;INNSBRUCK; A-6020; AUSTRIA (AT) GEHRER E ET AL 'Synthesis of masked glyoxal monoxime and 1-hydroxylaminoacetaldehyde derivatives'
- TETRAHEDRON LETT. (TELEAY,00404039);87; VOL.28 (9); PP.989-92, QUEEN MARY COLL.;DEP. CHEM.; LONDON; E1 4NS; UK (GB) SIMPKINS N S 'Preparation and reactions of a vinyl carbanion derived from an oxygenated vinyl sulfone'
- BULL. SOC. CHIM. FR. (BSCFAS,00378968);83; (1-2, PT. 2); PP.41-5, UNIV. POITIERS;LAB. CHIM. ORGANOMETALL.; POITIERS; 86022; FR. (FR) BARBOT F ET AL 'Preparation d'acétals fonctionnels'

## Beschreibung

Die vorliegende Erfindung betrifft ein Verfahren zur Herstellung von heterosubstituierten Acetalen durch Umsetzung von heterosubstituierten Vinylverbindungen mit Alkylnitriten in Gegenwart von Palladium in metallischer oder gebundener Form.

Heterosubstituierte Acetale der weiter unten genannten Formel (I) sind als Ausgangsmaterialien zur Synthese weiterer organischer Verbindungen, wie Arzneimittel, Pflanzenschutzmittel oder Farbstoffe, geeignet. So laßt sich beispielsweise 2,2-Dimethoxy-ethylphosphonsäure-diethylester zur Kettenverlängerung von Ketonen und Aldehyden einsetzen (Org. Synth. 53 (1973), 44-48). Die Umacetalisierung dieser Verbindung führt weiterhin zu phosphorhaltigen Polymeren (J. Polym. Sci. 22 (1984), 3335-3342; J. Polym. Sci. A 26 (1988), 2997-3014); solche Polymere werden in den Bereichen Flammschutzmittel, Hydrometallurgie und Membrantechnik eingesetzt.

Phenylsulfonyl-acetaldehyd-diethylacetal wird gemäß DE-OS 34 19 750 zu Vorprodukten für die Herstellung von Pyrethroiden umgesetzt.

Phenoxysulfonyl-acetaldehyd-dialkylacetal läßt sich zu 1,2-Benzoxathiin-2,2-dioxid umsetzen, welches gemäß EP 128 116 und EP 337 947 zu Herbiziden umgesetzt werden kann.

In der Literatur ist am Beispiel von Einzelfallen die Herstellung von Acetalen durch Umsetzung der zugrundeliegenden Olefine mit Alkylnitriten in Gegenwart von Palladiumsalzen beschrieben. So wird beispielsweise in EP 55 108 die Oxidation von Verbindungen, wie Ethen, Propen, Butylen, Cyclohexen, Acrylsäureestern und Acrylnitril, beschrieben. Die hierbei erzielten Ausbeuten sind allerdings gering, so daß bei einer eventuellen technischen Nutzung mit einem großen Aufwand zur Produktisolierung sowie zur Katalysator- und Lösungsmittelrückführung gerechnet werden muß. Insbesondere kann die in EP 55 108 vorgeschlagene Gasphasenreaktion nicht zur Herstellung von Acetalen der weiter unten genannten Formel (I) angewandt werden, da bei der hierzu erforderlichen hohen Temperatur mit dem Auftreten von Zersetzungs- und anderen Folgereaktionen gerechnet werden muß.

In J. Heterocyclic Chem. 29 (1992), 1625 wird die Umsetzung von 2-Nitro-styrolen mit Alkylnitriten in flüssiger Phase zu den entsprechenden Acetalen unter gleichzeitigem Zusatz von Sauerstoff beschrieben. Dabei können jedoch zündfähige Gemische entstehen, was insbesondere bei einer technischen Durchführung zu einem höheren Aufwand für die Bereitstellung der notwendigen Sicherheitsmaßnahmen führt.

In Synthesis 1997, 633 wird die Umsetzung von Ethylvinylether mit Ethylnitrit in Gegenwart von Bortrifluorid beschrieben, die jedoch lediglich zu einem Gemisch von Verbindungen führt.

Die Literatur enthält somit Verfahren, mit denen nicht substituierte oder an mindestens einem C-Atom mit einem weiteren C-Atom mono- oder disubstituierte Olefine umgesetzt werden können; die Literatur enthält insbesondere keine befriedigenden Verfahren, mit denen heterosubstituierte Vinylverbindungen zu Acetalen umgesetzt werden können. Wegen der vielfältigen Einsetzbarkeit heterosubstituierter Acetale bestand jedoch ein Bedürfnis, Verfahren zu deren Herstellung bereitzustellen.

Es wurde nun gefunden, daß Vinylverbindungen der weiter unten genannten Formel (II) überraschenderweise in hohen Ausbeuten durch Reaktion mit Alkylnitriten unter den Bedingungen des erfindungsgemäßen Verfahrens zu Acetalen der Formel (I) umgesetzt werden können.

Die Erfindung betrifft ein Verfahren zur Herstellung von heterosubstituierten Acetalen der Formel 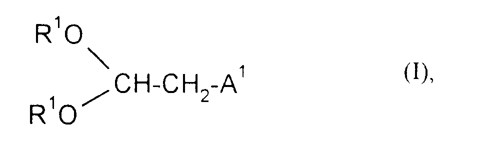 in der
- R¹: für geradkettiges oder verzweigtes C₁-C₈-Alkyl, bevorzugt C₁-C₂-Alkyl, besonders bevorzugt Methyl steht und
- A¹: ein Heteroatom aus der Gruppe von Silicium, Sauerstoff, Schwefel, Selen, Tellur, Stickstoff und Phosphor darstellt, das im Rahmen seiner Bindungsfähigkeit einen oder mehrere gleiche oder verschiedene Substituenten aus der Gruppe von doppelt gebundenem Sauerstoff, C₁-C₈-Alkoxy, C₁-C₈-Alkyl, Aryloxy, substituiertem Aryloxy, Aryl und substituiertem Aryl trägt und zusätzlich eine positive Ladung tragen kann,
das dadurch gekennzeichnet ist, daß man Vinylverbindungen der Formel

CH₂=CH-A¹ (II),

in der
- A¹: die obige Bedeutung hat,
in Gegenwart von Palladium in metallischer oder gebundener Form und in Alkoholen oder Ethern oder Gemischen mehrerer von ihnen als Reaktionsmedium bei 0 bis 120°C, bevorzugt 40 bis 80°C, mit Alkylnitriten der Formel

R¹-ONO (III),

in der
- R¹: die obige Bedeutung hat,
umsetzt. Geradkettiges oder verzweigtes C₁-C₈-Alkyl ist beispielsweise Methyl, Ethyl, Propyl, Isopropyl, Butyl, Isobutyl, tert.-Butyl, die isomeren Pentyle, Hexyle, Heptyle und Octyle. Bevorzugtes Alkyl ist Methyl oder Ethyl, besonders bevorzugt ist Methyl.

Geradkettiges oder verzweigtes C₁-C₈-Alkoxy ist beispielsweise Methoxy, Ethoxy, Propoxy, Isopropoxy, Butoxy, Isobutoxy, tert.-Butoxy, die isomeren Pentyloxy, Hexyloxy, Heptyloxy und Octyloxy. Bevorzugt ist C₁-C₄-Alkoxy, besonders bevorzugt Methoxy und Ethoxy.

Aryl hat 6 bis 12 C-Atome und ist beispielsweise Phenyl, Biphenylyl oder Naphthyl. Aryl kann 1 bis 3 mal gleichsinnig oder verschiedensinnig durch Methyl, Ethyl, Methoxy, Ethoxy, Chlor oder Brom substituiert sein. Aryloxy hat gleichfalls 6 bis 12 C-Atome und ist in unsubstituierter oder substituierter Form von beschriebenen Aryl abgeleitet.

A¹ ist ein Heteroatom aus der Gruppe von Silicium, Sauerstoff, Schwefel, Selen, Tellur, Stickstoff und Phosphor, bevorzugt aus der Gruppe von Silicium, Sauerstoff, Schwefel, Stickstoff und Phosphor und besonders bevorzugt aus der Gruppe von Schwefel und Phosphor.

Bevorzugte Vinylverbindungen sind demnach solche der Formel

CH₂=CH-A² (IV)

mit der Bedeutung von A² als einem substituierten Heteroatom aus der Gruppe von Silicium, Sauerstoff, Schwefel, Stickstoff und Phosphor.

Besonders wichtige Vinylverbindungen sind solche aus der Gruppe von

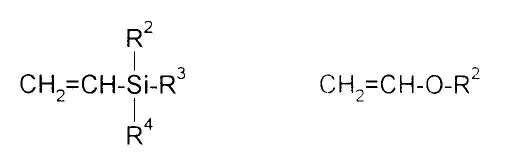

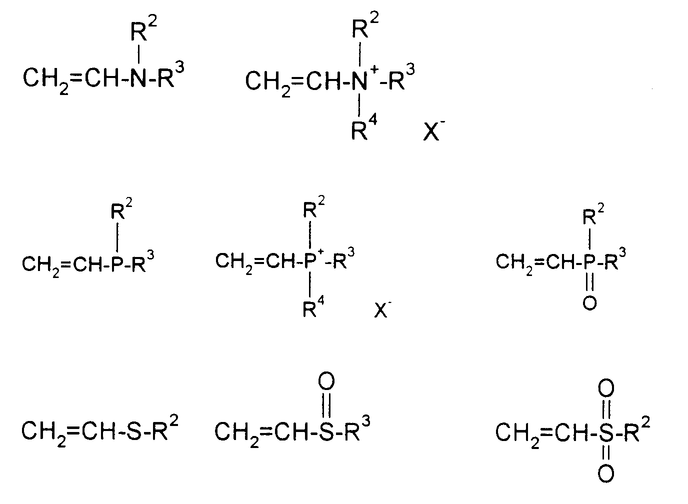

In diesen besonders wichtigen Vinylverbindungen bedeuten R², R³ und R⁴ unabhängig voneinander geradkettiges oder verzweigtes C₁-C₈-Alkoxy, C₁-C₈-Alkyl, C₆-C₁₂-Aryloxy oder C₆-C₁₂-Aryl, wobei Aryl bzw. Aryloxy in der obigen Weise substituiert sein können. Für den Fall, daß das Heteroatom eine positive Ladung trägt, wie dies aus den obigen Formulierungen ersichtlich ist, enthält eine solche Verbindung ein Anion X⁻ in der Bedeutung von Chlorid, Bromid, ½ Sulfat, Nitrat, Acetat, Trifluoracetat, Formiat, Propionat oder Benzoat. Aus der Reihe der oben formelmäßig aufgelisteten besonders wichtigen Vinylverbindungen sind die folgenden bevorzugt:

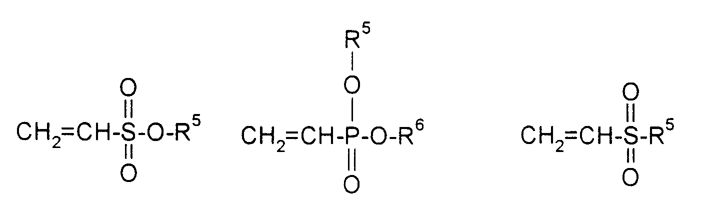

In diesen Formeln bedeuten R⁵ und R⁶ unabhängig voneinander Methyl, Ethyl oder Phenyl.

Palladium kann in metallischer oder gebundener Form eingesetzt werden. Beim Einsatz von Palladium in metallischer Form kann dies während der erfindungsgemäßen Reaktion mindestens teilweise in die gebundene Form übergehen. In bevorzugter Form wird Palladium jedoch in gebundener Form eingesetzt. Die gebundene Form umfaßt einfache Salze und Komplexsalze sowie Komplexverbindungen, in denen das Palladium nullwertig ist. Solche Palladiumsalze sollen im Reaktionsgemisch ganz oder teilweise löslich sein. Als Palladiumsalze seien beispielsweise genannt: Palladiumchlorid, Palladiumbromid, Palladiumacetat, Palladium-trifluoracetat, Palladiumsalze organischer Carbonsäuren, wie Propionsäure, Benzoesäure oder andere aliphatische oder aromatische Carbonsäuren, Palladiumsalze der Heteropolysäuren, insbesondere Palladiumsalze der von Vanadium, Molybdän, Niob und Wolfram abgeleiteten Heteropolysäuren. In bevorzugter Weise kommen die genannten Palladiumhalogenide, insbesondere Palladiumchlorid in Frage. Palladium in gebundener Form, insbesondere Palladium in Form der genannten Salze kann ergänzt werden durch Salze anderer Metalle, wie der Alkali- oder Erdalkalimetalle oder Salze des Ammoniums oder der verschieden hoch substituierten Amine. Als Anionen für solche Salze kommen die auch für das Palladium obengenannten in Frage. Viele dieser Salze bilden Komplexe mit Palladium in gebundener Form, so daß sie auch exemplarisch für die obengenannten Komplexsalze des Palladiums stehen. In bevorzugter Form sind solche ergänzenden Salze Lithiumchlorid oder Natriumchlorid, bevorzugt Lithiumchlorid.

Als Alkohole und Ether zur Durchführung des erfindungsgemäßen Verfahrens kommen C₁-C₄-Alkanole, wie Methanol, Ethanol, Propanol, Isopropanol, Butanol, Isobutanol oder tert.-Butanol, sowie Ethylenglykol, 1,2- oder 1,3-Propylenglykol oder 1,2-, 1,3-, 1,4- oder 2,3-Butylenglykol, deren Ether oder Halbether miteinander, wie Ethylenglykol-Dimethylether oder Diethylenglykol sowie Dioxan oder Tetrahydrofuran in Frage. Ether dieser Stoffe untereinander umfassen weiterhin Verbindungen, wie Diisopropylether, Dibutylether, Diethylenglykolmono-bzw. -diethylether. Aus Gründen der Vereinfachung der Reaktionsführung und der Aufarbeitung ist das Arbeiten in einem der genannten C₁-C₄-Alkanole bevorzugt. Besonders bevorzugt ist das Arbeiten in dem Alkanol, das dem zur erfindungsgemäßen Umsetzung benutzten Alkylnitrit zugrunde liegt.

Als Alkylnitrit sei beispielsweise Methylnitrit, Ethylnitrit, Propylnitrit, Isopropylnitrit, Butylnitrit oder Isobutylnitrit genannt.

Das erfindungsgemäße Verfahren wird bei einer Temperatur im Bereich von 0 bis 120°C, bevorzugt von 40 bis 80°C durchgeführt. Der Druck, unter dem die erfindungsgemäße Umsetzung durchgeführt wird, ist nicht kritisch und kann im Bereich von 5·10⁴-1,0·10⁶ Pa (0,5-10 bar), bevorzugt von 10·10⁵-6·10⁵ Pa (1,0-6 bar), besonders bevorzugt von 10⁵-5·10⁵ Pa (1-5 bar) liegen. Es ist jedoch auch möglich, bei höheren oder tieferen Drücken zu arbeiten.

Zur Reaktionsdurchführung kann beispielsweise das Palladium in metallischer oder gebundener Form zunächst in einem der genannten Lösungsmittel aufgeschlämmt oder ganz oder teilweise gelöst werden. Hierzu gibt man bei der angegebenen Temperatur die Vinylverbindung, gegebenenfalls im Gemisch mit einem Teil des vorgesehenen Lösungsmittels. Sodann wird das Alkylnitrit kontinuierlich oder absatzweise in flüssiger oder in gasförmiger Form zugegeben. Man kann jedoch auch die Zugabe der Vinylverbindung und des Alkylnitrits simultan durchführen. Im Falle des bevorzugt eingesetzten Methylnitrits wird dieses zweckmäßigerweise im Gemisch mit einem Inertgas in die Reaktionslösung eingeleitet. Als Inertgas kommt beispielsweise Stickstoff, Argon oder Kohlendioxid, bevorzugt Stickstoff, in Frage. Auch im Fall der Verwendung anderer Alkylnitrite kann es sinnvoll sein, beispielsweise aus sicherheitstechnischen Überlegungen, in Gegenwart eines Inertgases zu arbeiten. Die Menge des Lösungsmittels beträgt 1 bis 100 mol, bevorzugt 5 bis 40 mol, bezogen auf 1 mol eingesetzte Vinylverbindung.

Das molare Verhältnis der Vinylverbindung (II) zum Alkylnitrit (III) beträgt 1:1 bis 1:10, bevorzugt 1:1,5 bis 1:5.

Die Menge des Palladium in metallischer oder gebundener Form beträgt, gerechnet als Metall, 0,001 bis 0,2 g-Atom, bevorzugt 0,001 bis 0,1 g-Atom, bezogen auf 1 mol Vinylverbindung.

Die Isolierung und Aufarbeitung der Reaktionsprodukte erfolgt in einer dem Fachmann geläufigen Art, beispielsweise durch Destillation des Lösungsmittels und anschließende Feindestillation des Reaktionsproduktes unter vermindertem Druck, durch Kristallisation oder durch Kombination dieser Maßnahmen sowie gegebenenfalls durch Chromatographie.

### Beispiele

### Beispiel 1

Ein Gemisch aus 200 ml Methanol, 0,4 g (2,3 mmol) Palladiumchlorid und 0,2 g (4,7 mmol) Lithiumchlorid wurde auf 60°C erwärmt.

Anschließend begann man mit der kontinuierlichen Einleitung eines Gasstroms aus 10 l/h Stickstoff und 0,2 mol/h Methylnitrit.

Nach 15 min ließ man innerhalb 10 Minuten 32,8 g (0,2 mol) Vinylphosphonsäurediethylester zutropfen.

Während weiterer 2 Stunden wurde Methylnitrit im Stickstoffstrom eingeleitet, so daß insgesamt ca. 0,5 mol Methylnitrit verbraucht wurden.

Nach Beendigung der Einleitung von Methylnitrit wurde 1 Stunde lang im Stickstoffstrom nachgerührt.

Von der Lösung wurde Methanol abdestilliert, der Rückstand wurde im Vakuum destilliert.

Es wurden 38,2 g 2,2-Dimethoxy-ethylphosphonsäurediethylester erhalten (85 % d.Th.). Siedepunkt: 90-92°C/100 Pa (1 mbar).

### Beispiel 2

Ein Gemisch aus 200 ml Methanol, 0,25 g (1,4 mmol) Palladiumchlorid und 0,125 g (2,9 mmol) Lithiumchlorid wurde auf 60°C erwärmt.

Anschließend begann man mit der kontinuierlichen Einleitung eines Gasstroms aus 10 l/h Stickstoff und 0,1 mol/h Methylnitrit.

Nach 15 min ließ man 8,4 g (0,046 mol) Vinylsulfonsäurephenylester, gelöst in 20 ml Methanol, zutropfen.

Während weiterer 2 Stunden wurde Methylnitrit eingeleitet, so daß insgesamt ca. 0,2 mol Methylnitrit verbraucht wurden.

Nach Beendigung der Einleitung von Methylnitrit wurde 1 Stunde lang im Stickstoffstrom nachgerührt.

Von der Lösung wurde Methanol abdestilliert, der Rückstand wurde im Vakuum destilliert.

Man erhielt 9,1 g 2,2-Dimethoxyethyl-sulfonsäurephenylester (81 % d.Th.). Siedepunkt: 135°C/50 Pa (0,5 mbar).

### Beispiel 3

In 240 ml Methanol wurden unter Erwärmen auf 60°C 400 mg (2,3 mmol) Palladiumchlorid und 200 mg (4,7 mmol) Lithiumchlorid gelöst.

Anschließend wurde ein kontinuierlicher Gasstrom aus 10 l/h Stickstoff und 0,2 mol/h Methylnitrit eingeleitet.

Nach 15 Minuten ließ man eine Lösung von 30 g (0,18 mol) Phenylvinylsulfon in 60 ml Methanol zutropfen.

Über 2 Stunden lang wurde weiter Methylnitrit eingeleitet, so daß insgesamt ca. 0,4 mol Methylnitrit verbraucht wurden.

Von der Lösung wurde Methanol abdestilliert, der Rückstand wurde im Vakuum destilliert.

Man erhielt 31,6 g (0,14 mol) 2,2-Dimethoxy-ethylphenylsulfon (77 % d.Th.). Siedepunkt: 139-140°C/70 Pa (0,7 mbar).

## Patentansprüche

1. Verfahren zur Herstellung von heterosubstituierten Acetalen der Formel 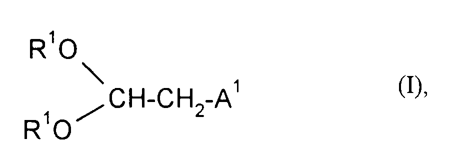 in der
R¹ für geradkettiges oder verzweigtes C₁-C₈-Alkyl, bevorzugt C₁-C₂-Alkyl, besonders bevorzugt Methyl steht und
A¹ ein Heteroatom aus der Gruppe von Silicium, Sauerstoff, Schwefel, Selen, Tellur, Stickstoff und Phosphor darstellt, das im Rahmen seiner Bindungsfähigkeit einen oder mehrere gleiche oder verschiedene Substituenten aus der Gruppe von doppelt gebundenem Sauerstoff, C₁-C₈-Alkoxy, C₁-C₈-Alkyl, Aryloxy, substituiertem Aryloxy, Aryl und substituiertem Aryl trägt und zusätzlich eine positive Ladung tragen kann,
dadurch gekennzeichnet, daß man Vinylverbindungen der Formel
CH₂=CH-A¹ (II),
in der
A¹ die obige Bedeutung hat,
in Gegenwart von Palladium in metallischer oder gebundener Form und in Alkoholen oder Ethern oder Gemischen mehrerer von ihnen als Reaktionsmedium bei 0 bis 120°C, bevorzugt 40 bis 80°C, mit Alkylnitriten der Formel
R¹-ONO (III),
in der
R¹ die obige Bedeutung hat,
umsetzt.

2. Verfahren nach Anspruch 1, dadurch gekennzeichnet, daß eine Vinylverbindung der Formel
CH₂=CH-A² (IV)
eingesetzt wird, in der
A² ein substituiertes Heteroatom aus der Gruppe von Silicium, Sauerstoff, Schwefel, Stickstoff und Phosphor darstellt, das zusätzlich eine positive Ladung tragen kann.

3. Verfahren nach Anspruch 1, dadurch gekennzeichnet, daß Vinylverbindungen aus der Gruppe von 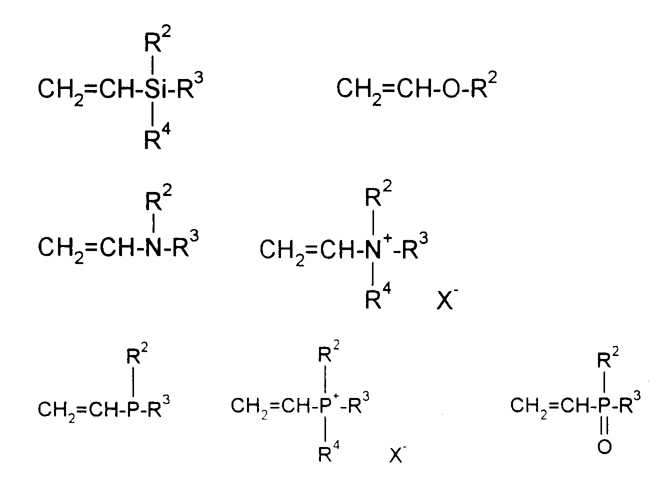 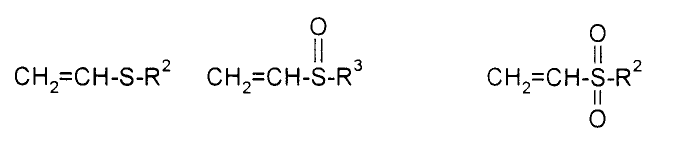 eingesetzt werden, worin
R², R³ und R⁴ unabhängig voneinander geradkettiges oder verzweigtes C₁-C₈-Alkoxy, C₁-C₈-Alkyl, C₆-C₁₂-Aryloxy, substituiertes C₆-C₁₂-Aryloxy, C₆-C₁₂-Aryl oder substituiertes C₆-C₁₂-Aryl darstellen und
X⁻ Chlorid, Bromid, ½ Sulfat, Nitrat, Acetat, Trifluoracetat, Formiat, Propionat oder Benzoat bedeutet.

4. Verfahren nach Anspruch 3, dadurch gekennzeichnet, daß Vinylverbindungen aus der Gruppe von 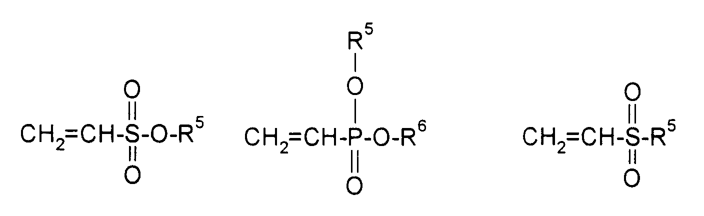 eingesetzt werden, worin
R⁵ und R⁶ unabhängig voneinander Methyl, Ethyl oder Phenyl bedeuten.

5. Verfahren nach Anspruch 1, dadurch gekennzeichnet, daß Palladium in gebundener Form, bevorzugt als Palladiumhalogenid, besonders bevorzugt als Palladiumchlorid eingesetzt wird, das durch Zusatz eines weiteren Salzes in eine komplex gebundene Form übergeführt werden kann.

## Claims

1. Process for the preparation of heterosubstituted acetals of the formula 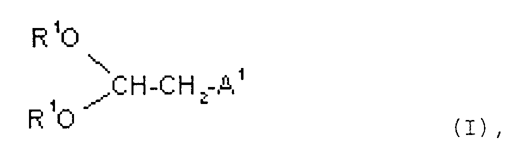 in which
R¹ represents straight-chain or branched C₁-C₈-alkyl, preferably C₁-C₂-alkyl, particularly preferably methyl, and
A¹ represents a heteroatom from the group consisting of silicon, oxygen, sulphur, selenium, tellurium, nitrogen and phosphorus, which, within the scope of its bonding ability, carries one or more identical or different substituents from the group consisting of double-bonded oxygen, C₁-C₈-alkoxy, C₁-C₈-alkyl, aryloxy, substituted aryloxy, aryl and substituted aryl, and may in addition carry a positive charge,
characterized in that vinyl compounds of the formula
CH₂=CH-A¹ (II),
in which
A¹ has the above meaning,
are reacted, in the presence of palladium in metallic or bonded form and in alcohols or ethers or mixtures of two or more of them as reaction medium at from 0 to 120°C, preferably from 40 to 80°C, with alkyl nitrites of the formula
R¹-ONO (III),
in which
R¹ has the above meaning.

2. Process according to Claim 1, characterized in that a vinyl compound of the formula
CH₂=CH-A² (IV)
is employed in which
A² represents a substituted heteroatom from the group consisting of silicon, oxygen, sulphur, nitrogen and phosphorus, which may in addition carry a positive charge.

3. Process according to Claim 1, characterized in that vinyl compounds from the group consisting of 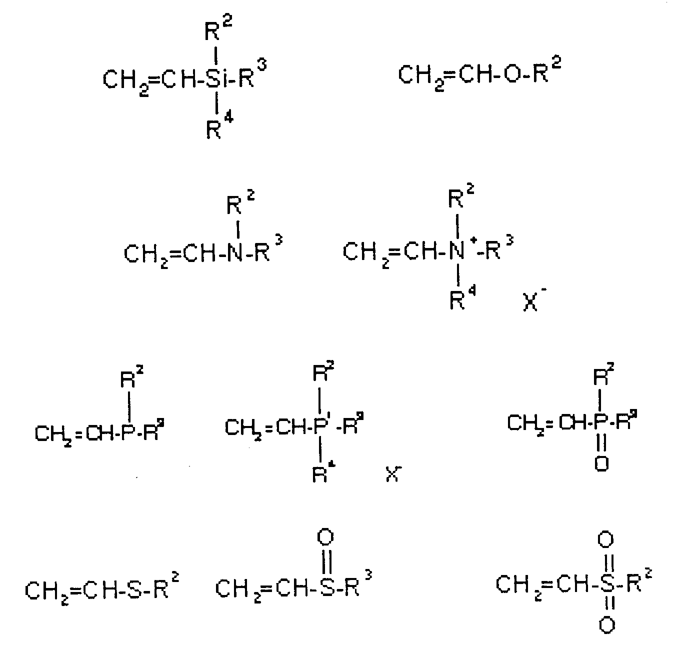 are employed in which
R², R³ and R⁴ represent independently of one another straight-chain or branched C₁-C₈-alkoxy, C₁-C₈-alkyl, C₆-C₁₂-aryloxy, substituted C₆-C₁₂-aryloxy, C₆-C₁₂-aryl or substituted C₆-C₁₂-aryl, and
X⁻ denotes chloride, bromide, ½ sulphate, nitrate, acetate, trifluoroacetate, formate, propionate or benzoate.

4. Process according to Claim 3, characterized in that vinyl compounds from the group consisting of 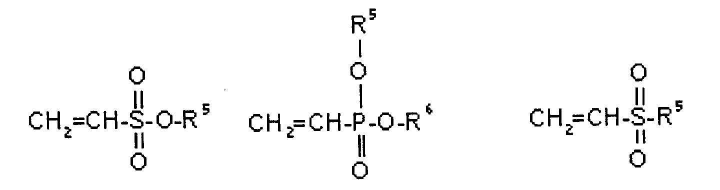 are employed in which
R⁵ and R⁶ denote independently of one another methyl, ethyl or phenyl.

5. Process according to Claim 1, characterized in that palladium is employed in bonded form, preferably as palladium halide and particularly preferably as palladium chloride, which may be converted into a complexed form by addition of a further salt.

## Revendications

1. Procédé de préparation d'acétals hétérosubstitués de formule : 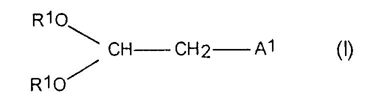 dans laquelle :
R¹ représente un alcoyle en C₁-C₈ linéaire ou ramifié, de préférence un alcoyle en C₁-C₂, de manière particulièrement préférée méthyle, et
A¹ représente un hétéroatome du groupe du silicium, de l'oxygène, du soufre, du sélénium, du tellure, de l'azote et du phosphore, qui peut porter, dans le cadre de sa capacité de liaison, un ou plusieurs substituants identiques ou différents parmi le groupe de l'oxygène doublement lié, alcoxy en C₁-C₈, alcoyle en C₁-C₈, aryloxy, aryloxy substitué, aryle et aryle substitué et en outre, peut porter une charge positive,
caractérisé en ce que l'on transforme le composé vinylique de formule
CH₂=CH-A¹ (II),
dans laquelle :
A¹ a la signification donnée ci-dessus,
en présence de palladium sous forme métallique ou liée et dans un alcool ou un éther ou un mélange de plusieurs de ceux-ci comme milieu de réaction, entre 0 et 120°C, de préférence 40 et 80°C, avec un nitrite d'alcoyle de formule :
R¹-ONO (III),
dans laquelle :
R¹ a la signification donnée ci-dessus.

2. Procédé suivant la revendication 1, caractérisé en ce que l'on met en oeuvre un composé vinylique de formule :
CH₂=CH-A² (IV)
dans laquelle A² représente un hétéroatome substitué du groupe du silicium, de l'oxygène, du soufre, de l'azote et du phosphore, qui peut porter en outre, une charge positive.

3. Procédé suivant la revendication 1, caractérisé en ce que l'on met en oeuvre les composés vinyliques du groupe : 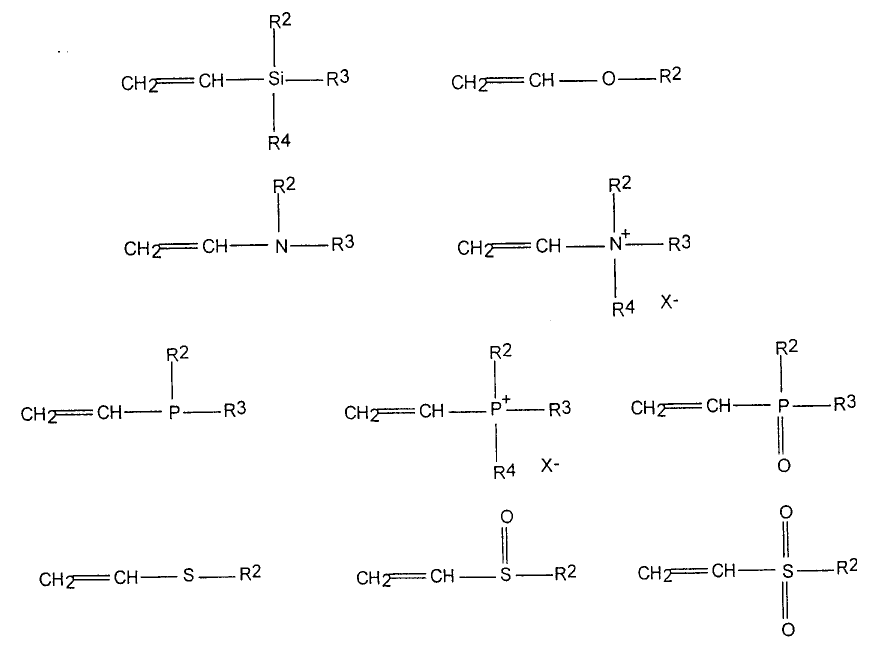
où R², R³ et R⁴ représentent indépendamment l'un de l'autre, alcoxy en C₁-C₈, alcoyle en C₁-C₈ linéaires ou ramifiés, aryloxy en C₆-C₁₂, aryloxy en C₆-C₁₂ substitué, aryle en C₆-C₁₂, aryle en C₆-C₁₂ substitué, et
X⁻ représente chlorure, bromure, 1/2 sulfate, nitrate, acétate, trifluoroacétate, formiate, propionate ou benzoate.

4. Procédé suivant la revendication 3, caractérisé en ce que l'on met en oeuvre les composés vinyliques du groupe : 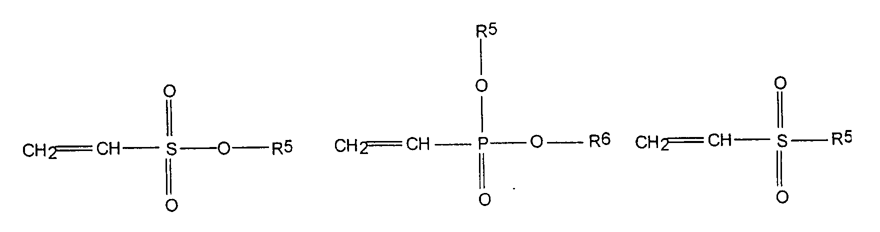 où R⁵ et R⁶ représentent indépendamment l'un de l'autre, méthyle, éthyle ou phényle.

5. Procédé suivant la revendication 1, caractérisé en ce que le palladium est mis en oeuvre sous forme liée, de préférence sous forme d'halogénure de palladium, de manière particulièrement préférée, de chlorure de palladium, lequel peut être converti en une forme liée complexe par addition d'un autre sel.
